Europäisches Patentamt

European Patent Office

Office européen des brevets

(11) Publication number: **0 396 744 B1**

# EUROPEAN PATENT SPECIFICATION

(45) Date of publication of patent specification: **03.11.93**

(21) Application number: **88906077.8**

(22) Date of filing: **08.07.88**

(86) International application number:
**PCT/JP88/00686**

(87) International publication number:
**WO 89/00425 (26.01.89 89/03)**

The file contains technical information submitted after the application was filed and not included in this specification

(51) Int. Cl.⁵: **A61K 35/72**, A61K 35/74, C12N 1/16, C12N 1/20

(54) **LIPID PEROXIDE CONTENT REDUCER.**

(30) Priority: **09.07.87 JP 169744/87**

(43) Date of publication of application:
**14.11.90 Bulletin 90/46**

(45) Publication of the grant of the patent:
**03.11.93 Bulletin 93/44**

(84) Designated Contracting States:
**DE FR GB IT**

(56) References cited:
**EP-A- 0 181 170**
**JP-A-62 103 023**
**JP-A-62 103 026**
**JP-A-63 130 538**
**US-A- 4 251 519**

**CHEMICAL ABSTRACTS, vol. 96, no. 15, 2nd April 1982, page 330, abstract no. 118634v, Columbus, Ohio, US; Y. IWAMOTO et al.: "Superoxide dismutase activity of lac-**

tobacilli"

(73) Proprietor: **KABUSHIKI KAISYA ADVANCE**
**5-7, Nihonbashi Kobuna-cho**
**Chuo-ku Tokyo 103(JP)**

(72) Inventor: **ISHIHARA, Kazuoki**
**2-13-7, Uchikanda**
**Chiyoda-ku**
**Tokyo 101(JP)**
Inventor: **ITO, Masonori Haitsu Ishibiki 302**
**1-13-6, Ishibiki**
**Kanazawa-shi**
**Ishikawa 920(JP)**

(74) Representative: **Cohausz & Florack Patentan-wälte**
**Postfach 14 01 61**
**Schumannstrasse 97**
**D-40237 Düsseldorf (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

TECHNICAL FIELD

The present invention relates to a lipid peroxide reducing agent having lactobacterium and yeast cells as effective components. More specifically, it relates to a lipid peroxide reducing agent which contains, as an effective component, the cells of microorganisms belonging to the genus Enterococcus, Lactobacillus, and/or Saccharomyces. In the present specification, the term "lipid peroxide" generally denotes peroxides of lipids, such as peroxides of linolenic acid, oleic acid or arachidonic acid.

BACKGROUND ART

It is known that the lipid peroxides contained in food obstruct the membrane of the digestive tract and that some are taken into the body and have various effects. It has been reported, for example, that when lipid peroxides are produced in cell walls, which contain large amounts of the unsaturated fatty acids in the body, they cause a drop in the activity of various enzymes, the properties of the membrane deteriorate, for example, red blood cells easily undergo hemolysis or the vein walls harden, and morphological changes occur in various tissues. These various phenomena overlap and are considered to result in practice in hardening of the arteries, a decline in the enzyme function, retinopathy, cataracts, diabetes, and other declines in function due to aging.

At the present time, to alleviate and cause recovery from such symptoms, use is primarily made of tocophenol, ascorbic acid, and other antioxidants. These, however, are extremely easily oxidized in themselves and problems occur in maintaining their efficacy. Further, since they are food additives, an intake thereof alone is not desirable.

In consideration of the above, the present inventors engaged in indepth research, and as a result, discovered that certain types of lactobacterium and yeast have the effect of significantly reducing the lipid peroxides, and thus arrived at the present invention.

Accordingly, the object of the present invention is to provide a bacterial cell product capable of reducing the lipid peroxides in food and the digestive tract. Another object of the present invention is to provide a bacterial cell product which remains efficacious over a long period by maintaining the agent in a dried state without observation of a weakening of the lipid peroxide reductive power. Further, the object of the present invention is to provide a bacterial cell product which can be ingested alone by itself as a food.

These objects are achieved with a lipid peroxide reducing agent containing as an effective component at least one substance selected from viable cells, dead cells and an insoluble component thereof obtained from Enterococcus faecium AD 1060 (FERM BP-1926), Lactobacillus salivarius AD 003 (FERM BP-1927), E. faecalis AD 9001 (FERM BP-297), or Saccharomyces cerevisiae 155-77 (FERM BP-1928).

From another aspect, the present invention relates to a novel strain having a lipid peroxide reducing activity, which is at least one strain selected from Enterococcus faecium AD 1060 (FERM BP-1926), Lactobacillus salivarius AD 0003 (FERM BP-1927), and Saccharomyces cerevisiae 155-77 (FERM BP-1928).

The lipid peroxide reducing agent of the invention may further contain a pharmaceutically acceptable carrier and may be provided in a dried form suitable for storage.

Chemical Abstracts 96 (1982), 118634v and JP-A-62 103 023 refer to superoxide dismutase, which is an enzyme that destroys superoxide anion radicals, represented by "-O$_2$ ". In contrast thereto according to the invention lipid peroxide reducing agents are provided, which act on "-O$_2$-". Having in mind the high specifity of enzymes the fact that a strain contains superoxide dismutase does not suggest that this strain has an activity to reduce a lipid peroxide.

BRIEF DESCRIPTION OF THE DRAWINGS

Figure 1 is a graph illustrating the result of Example 1, wherein the ordinate shows the cell concentration (mg/ml) and the abscissa shows the reduction in LPO (%).

Figure 2 is a flow chart of the procedural method of Example 2.

BEST MODE OF CARRYING OUT THE INVENTION

As stated above, the lipid peroxide reducing agent according to the present invention can be prepared from one or more microorganisms listed in the following Table 1.

Table 1

| No. | Name of strain | Deposition number |
|---|---|---|
| 1 | Enterococcus faecium AD1060 | FERM BP-1926 (FERM P-9414) |
| 2 | Enterococcus faecalis AD9001 | FERM BP-297 (FERM P-6625) |
| 3 | Lactobacillus salivarius AD0003 | FERM BP-1927 (FERM P-9415) |
| 4 | Saccharomyces cerevisiae 155-77 | FERM BP-1928 (FERM P-9416) |

In Table 1, the strains No. 1, No. 3 and No. 4 have been deposited since June 13, 1987 in the Fermentation Research Institute, Agency of Industrial Science and Technology, the Ministry of International Trade and Industry (1-3, Higashi 1 chome Yatabe-machi, Tsukuba-gun, Ibaraki-ken, 305, JAPAN) [hereinafter referred to as Bikoken (FRI)] under the domestic deposition (deposition numbers are indicated as FERM-P), and transferred since June 29, 1988 to FRI as the international depository authority under the Budapest Treaty (deposition numbers are indicated as FERM-BP). The strain No. 2 has been deposited at the FRI under the domestic system since July 15, 1982, and transferred to the international system since May 31, 1983.

The general microbiological characteristics of the microorganisms of the present invention are the same as those of known microorganisms belonging to the identical classes. That is, the general microbiological characteristics, cultivation methods and other properties correspond to those described in the following articles:

1) Bergey's Manual of Determinative Bacteriology, 8th ed., 490 - 509 (1974)

2) Int. J. Syst. Bact. 16, 114 (1966)

3) Poupard, J.A., Husain, I. and Norris, R.F: Bacteriol. Rev. 37, 136 - 165 (1973)

4) Mituoka, T. Jpn. J. Bacteriol. 24, 261 - 280 (1969)

5) Nogeikagaku Jikken Sho (Handbook of Experiments in Agricultural Chemistry), vol. 2 (1964), edited by Agricultural Chemistry Class, Agricultural Department, Kyoto University

6) Microbiol. Immunol. 25 (3), 257 - 269 (1981)

7) J. Clin. Pathol. 33 53 - 57 (1980)

8) J. General Microbiol., 128 713 - 720 (1982)

9) Applied Microbiol., 23 (6) 1131 - 1139 (1972)

10) Mitsuoka, T., Rinsho to Saikin (Clinical Medicine and Microorganism), 2 (3), (197)55 - (235)93, 1975

11) T. Watanabe, H. Shimohashi, Y. Kawai, M. Muti: 25 (3), 257 - 269, 1981

12) R.H. Deibel, D.E. Lake, C.F. Nieven. Jr: J. Bacteriol. 86, 1275 - 1282, 1963

Typical microbiological characteristics of the present strains listed in Table 1 are summarized in Table 2 through Table 6.

## Table 2

Enterococcus faecium AD1060
(FERM BP-1926)

| | |
|---|---|
| Gram strain | + |
| Catalase (in the presence of boiled blood) | - |
| Growth at 10°C | + |
| Growth at 45°C | + |
| Growth at 50°C | + |
| Growth at pH 9.6 | + |
| Growth in 6.5% NaCl | + |
| Growth in 40% bile | + |

| | |
|---|---|
| Growth in 1/4000 tellurious acid | − |
| Growth in 0.1% methylene blue milk | + |
| Fermentation of Carbohydrate | |
| Arabinose | + |
| Glycerol | − |
| Raffinose | + |
| Sorbitol | − |
| Lactose | + |
| Melezitose | − |
| Inulin | − |
| Esculin | + |
| Ammonia generation from arginine | + |
| Hydrolysis of hippuric acid | − |

## Table 3

### Lactobacillus salivarius AD0003
### (FERM BP-1927)

| | |
|---|---|
| Shape | Bacillus |
| Gram strain | + |
| Gas generation from glucose | − |
| Growth at 15°C | − |
| Growth at 45°C | + |
| Fermentation of carbohydrate | |
| Arabinose | − |
| Xylose | − |
| Rhamnose | − |
| Ribose | − |
| Mannose | + |
| Fructose | + |

| | |
|---|---|
| Galactose | + |
| Sucrose | + |
| Maltose | + |
| Cellobiose | - |
| Lactose | + |
| Trehalose | + |
| Melibiose | + |
| Raffinose | + |
| Melezitose | - |
| Dextrin, starch | N. D. |
| Mannitol | + |
| Sorbitol | + |
| Esculin | + |
| Salicin | + |
| Amygdalin | - |

## Table 4

Saccharomyces cerevisiae 155-77
(FERM BP-1928)

| | |
|---|---|
| Shape | Egg-shaped |
| Proliferation | Budding |
| Sporogenesis | + |
| Fermentation | |
| Glucose | + |
| Fructose | + |
| Mannose | + |
| Galactose | + |
| Sucrose | + |
| Maltose | + |

| | |
|---|---|
| Lactose | - |
| Raffinose | + |
| Precipitated yeast | + |
| Yeast circle | - |
| Theca | - |
| Liquefaction of gelatin | - |
| Anabolism of sulfate | - |

## Table 5

Enterococcus faecalis AD9001
(FERM BP-297)

| | |
|---|---|
| Cell shape | Spherical |
| Gram stain | + |
| Hemolyzation | α |
| Growth at 10°C | + |
| Growth at 45°C | + |
| Growth at 50°C | - |
| Heat resistance at 60°C for 30 minutes | + |
| Growth in pH 9.6 medium | + |
| Capability of reducing methylene blue | + |
| Liquefaction of gelatin | - |
| Growth in medium containing 6.5% NaCl | + |
| Growth in medium containing 40% bile | + |
| Production of ammonia | + |
| Hydrolysis of hippuric acid | ± |
| Growth in medium containing tellurite | + |
| Growth in medium containing TTC* | + |
| Production of acid from carbon source | |

7

| | |
|---|---|
| Glucose | + |
| Esculin | + |
| Inulin | - |
| Lactose | + |
| Glycerol | + |
| Arabinose | - |
| Melezitose | + |
| Sorbitol | + |
| Serum (group antigens) | 'D |

(*2,3,5-triphenyltetrazolium chloride)

The microbial cells according to the present invention are prepared as follows:

The microbial cell is inoculated into the Rogosa broth medium as mentioned below (in the case of lactobacterium, i.e., the microorganisms belonging to the genus Enterococcus and Lactobacillus) or a broth medium containing 0.2% of ammonium sulfate, 0.2% of yeast extract, 2% of glucose and 0.1% of peptone (in the case of yeast, i.e., the microorganism belonging to the genus Saccaromices), and cultivated with shaking at 37°C for 16 - 24 hours for the former, or at 30°C for 24 - 48 hours for the latter. The microorganisms are harvested by centrifugation (1000 g, 10 min), suspended in purified water (1/100 volume of the medium), and then washed by centrifugation. The washed microorganism may be used as a viable cell. Alternatively, a dead cell can be used which is prepared by killing the cell with heating at 121°C for 10 minutes and then drying (freeze-drying or heat-drying). Further, an insoluble component may be used which is prepared by centrifuging the dead cell.

### Composition of Rogosa broth medium

| | |
|---|---|
| Trypticase | 10 g |
| Yeast extract | 5 g |
| Tryptose | 3 g |
| $K_2HPO_4$ | 3 g |
| $KH_2PO_4$ | 3 g |
| Sodium acetate | 1 g |
| Triammonium citrate | 2 g |
| Tween 80 | 1 g |
| Glucose | 20 g |
| L-cysteine hydrochloride | 0.2 g |
| Salt solution (*) | 5 ml |
| Distilled water | to 1 liter |

(pH 7, heat-sterilization at 121°C for 15 minutes)

(*) Salt solution:

| | |
|---|---|
| $MgSO_4-7H_2O$ | 11.5 g |
| $FeSO_4-7H_2O$ | 0.68 g |
| $MnSO_4-2H_2O$ | 2.4 g |
| Distilled water | 100 ml |

The screening of lactobacterium used in the present invention is carried out in accordance with the procedure disclosed in Watanabe, T., et al., Studies on streptococci, I. Distribution of fecal streptococci in man. Microbiol. Immunol. 25 257 - 269 (1981). That is, as mentioned in this literature, the feces obtained from healthy adults were smeared on KMN agar or LBS agar, and cultivated at 37°C for 48 - 72 hours under an aerobic condition. The formed colonies were counted and randomly isolated. In view of the colony type, catalase activity (negative), and gram stain (positive), coccus and bacillus were isolated. The isolates were identified and classified by examining physiological, biochemical, and serological properties.

The lipid peroxide reducing agent according to the present invention comprises the above-mentioned viable cells, dead cells, or the insoluble component, and optionally, a pharmaceutically acceptable carrier (for example, starch, crystalline cellulose, calcium carboxymethyl cellulose).

The lipid peroxide reducing agent according to the present invention may be taken up by a peroral administration, and the dose thereof is usually about 0.1 to about 50 mg/kg body weight (once). The agent may be used in the form of a pellet, tablet, granule or the like.

EXAMPLES

The present invention will now be further shown by the following examples.

The microbial cells used in the following Examples were prepared by the procedure explained as above. The lipid peroxide was prepared and measured in accordance with the following procedures.

Method of Preparation of Lipid Peroxides

Linolic acid was oxidized by the auto-oxidation method or the photosensitization oxidation method, then the peroxides separated out by thin layer chromatography (reference material: "Kasankashishitsu Jikkenho" (Experimental Methods for Lipid Peroxides), Masashi Kaneda, Nobuo Ueda ed., Ishiyaku Shuppan KK, Agric. Biol. Chem. 35, p. 33 to 39, 1971; supra, 45, p. 587 to 593, 1981, "Hanyou Eisei Shikenho to Kaisetsu" (General Sanitary Test Methods and Analysis", Japan Academy of Pharmacology ed., Nanzando, p. 33, "Shishitsu Bunsekiho Nyumon" (Introduction of Lipid Analysis Methods), Yasuhiko Fujino, Gakkai Shuppan Center, p. 100).

Method of Measurement of Lipid Peroxides

The measurement of the lipid peroxides was carried out by the TBA (thiobarbituric acid) method ("Nihon Ronen Igaku Zasshi" (Japan Journal of Geriatrics), Chikayuki Naito, Ken Yamanaka, 15, p. 187 to 191, 1978). The method is explained below.

Method

(1) Cause a reaction between the cells (given amounts) and lipid peroxide (LPO) solution (2 ml) in test tubes equipped with glass stoppers.
(2) Add 3.0 ml of 0.05N hydrochloric acid to the reaction solution (1).
(3) Add 3.0 ml of 0.67 percent TBA reagent and mix.
(4) Seal the test tube and heat with boiling water for 30 minutes.
(5) Place in ice-cooled water tank to quickly cool to room temperature.
(6) Add 2.0 ml of n-butanol and replacement-extract.
(7) Centrifuge (2500 rpm for 10 minutes), then take off the butanol layer (top layer) and perform colorimetric measurement at 535 nm.

The standard solution used was a 10 nmol/ml tetraethoxypropane methanol solution. To prevent interference with the TBA reaction product of the cells themselves, samples with no lipid peroxides also were measured. The value minus this value is designated as the TBA value.

The concentration (C) of lipid peroxides (nmol/ml) is found by the following formula:

$$C = \frac{\text{Absorbance of specimen}}{\text{Absorbance of standard solution}} \times 10 \ (nmol/ml)$$

In the following Examples, the artificial intestinal juice used was prepared by adding 250 ml of 0.2M potassium dihydrogen phosphate and 118 ml of 0.2N sodium hydroxide to purified water and adjusting the whole to 1000 ml.

Example 1 Amount of the Cells and LPO Reduction

One, 5, 10, 15, and 20 mg amounts of hot-water-treated-freeze-dried cells of Enterococcus faecium AD1060 (FERM BP-1926) were suspended in 2 ml portions of artificial intestinal juice having concentration of lipid peroxides of 125, 250, and 500 $\mu$g/ml to prepare experimental solutions having cell concentrations of 0.5, 2.5, 5.0, 7.5, and 10.0 mg/ml.

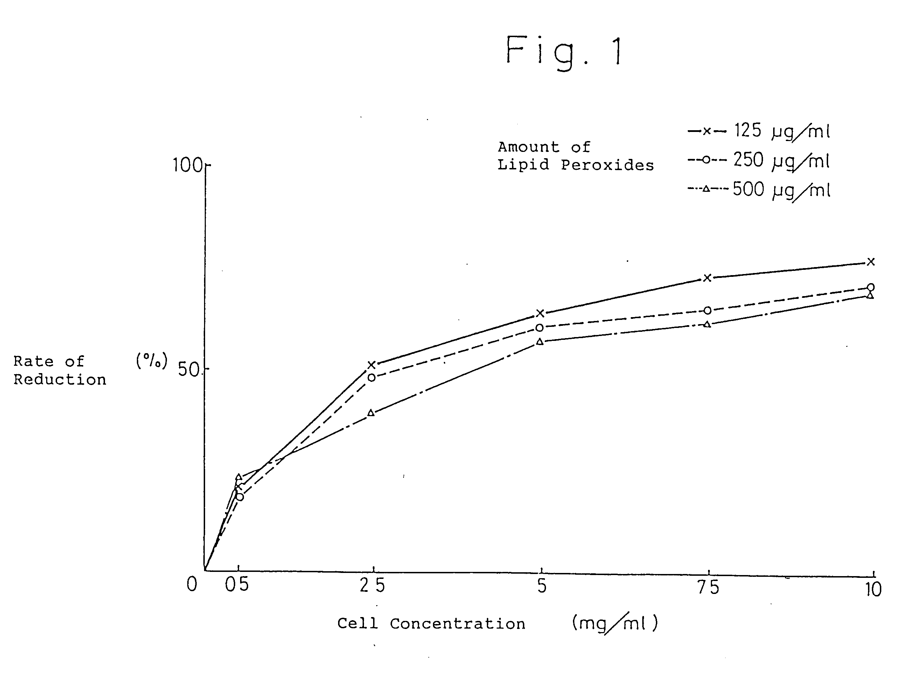

In accordance with the above-mentioned measurement method, the rate of reduction of lipid peroxides was found. The results, as shown in Table 6 and Fig. 1, show an effective reduction of lipid peroxides.

Table 6

| Cell concentration | Lipid peroxide concentration | | |
|---|---|---|---|
| | 125 $\mu$g/ml | 250 $\mu$g/ml | 500 $\mu$g/ml |
| 0.5 mg/ml | 21.5% | 18.7% | 22.9% |
| 2.5 mg/ml | 51.6% | 47.9% | 39.5% |
| 5.0 mg/ml | 64.5% | 60.7% | 57.6% |
| 7.5 mg/ml | 73.1% | 65.3% | 62.3% |
| 10.0 mg/ml | 77.1% | 70.8% | 69.7% |

Example 2 Reduction of LPO in Food by Cells

Ten, 30, 50 and 100 mg amounts of hot-water-treated and freeze-dried cells of Enterococcus faecium AD1060 (FERM BP-1926) were suspended in 1 g amounts of dressing, allowed to stand at room temperature for one hour, and then measured to find the LPO reduction rate using the TBA method.

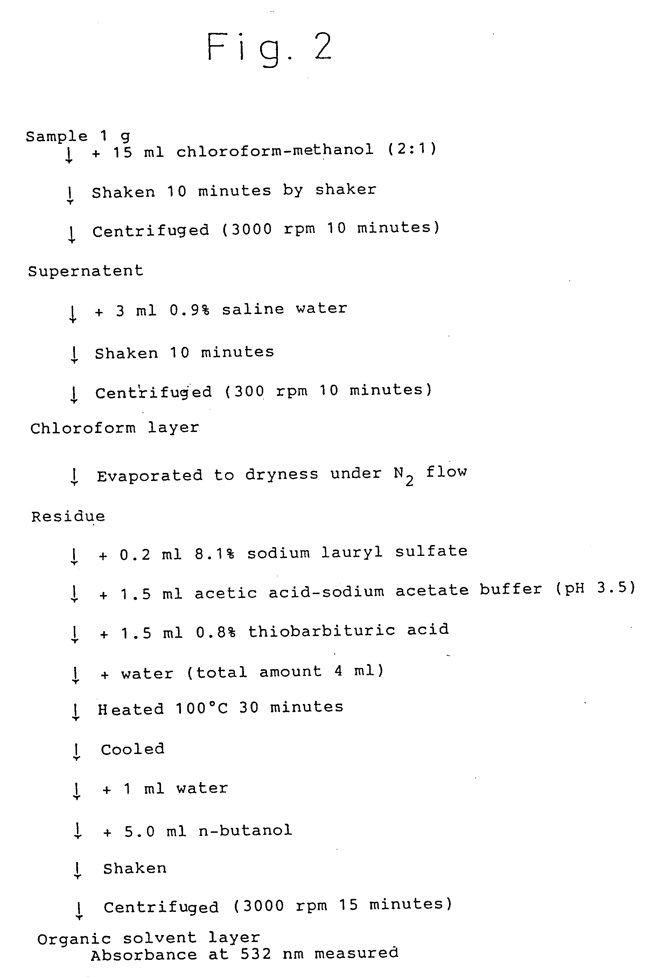

The TBA method used in the present Example is shown in Fig. 2. The results are listed in Table 7. As shown in Table 7, a reduction of 90.2 percent was observed with the addition of 100 mg of cells.

Table 7

| Cells (mg) | TBA value (nmol) | Reduction rate (%) |
|---|---|---|
| 0 | 29.5 | - |
| 10 | 13.8 | 53.2 |
| 30 | 8.4 | 71.5 |
| 50 | 5.0 | 83.1 |
| 100 | 2.9 | 90.2 |

Example 3 Ability to Reduce Lipid Peroxides After Treatment by Digestive Enzymes

To investigate the effects of the lipid peroxide reducing agent of the present invention in the digestive tract, pepsin, trypsin, thymotrypsin, and bovine bile powder were added to the heated and freeze-dried dead cells [Enterococcus faecium AD1060 (FERM BP-1926)] (hereinafter referred to as heat-dried dead cells) to study the ability to reduce the lipid peroxides. Further, an experiment was performed on the insoluble portions of the heat-dried dead cells, which are believed to contain particularly large amounts of the effective component of the agent of the present invention. As a control, the reduction rate of untreated heat-dried dead cells was found and used as 100. The results are shown in Table 8.

Pepsin Treatment

1) A 0.1 mg amount of pepsin (made by Wako Junyaku Kogyosha) was dissolved in 2 ml of 0.1M citrate buffer (pH 2.5) and 20 mg of heat-dried dead cells was suspended and treated at 37°C for hour hours. To this was added 1000 $\mu$g of lipid peroxides and then one hour later the amount of lipid peroxide was measured by the TBA method. A specimen prepared in the same way but without the addition of the cells was used as the control to find the rate of reduction of lipid peroxides (A). (A) was divided by the rate of reduction of lipid peroxides (B) in an artificial intestinal juice including 20 mg of untreated heat dried cells and 1000 $\mu$g of lipid peroxides and then multiplied by 100.

2) The above-mentioned pepsin treatment was performed for four hours and the centrifugation performed to collect the insoluble portion. To this was added 10 ml of distilled water and then a cycle of resuspension and centrifugation was repeated twice to wash the insoluble portion. This was freeze dried (the same results were obtained for the cells heat-dried at 80°C) and then added to 2 ml of an artificial intestinal juice including 1000 $\mu$g of lipid peroxides. After one hour, the amount of lipid peroxides was measured by the TBA method and the rate of reduction of lipid peroxides (C) found. (C) was divided by the above-mentioned (B) and multiplied by 100.

Trypsin Treatment and Chymotrypsin Treatment

Amounts of 0.1 mg of trypsin or chymotrypsin (made by Wako Junyaku Kogyosha) were dissolved in 2 ml portions of 0.1M phosphate buffer (pH 7.5) and then the rate of reduction of lipid peroxides found by the same process as the pepsin treatment.

Insoluble Portion of Heat-Dried Dead Cells

A 20 mg amount of heat-dried dead cells was suspended in 100 ml of water, kept at 60°C for 10 minutes, then centrifuged to collect the insoluble portion, and then the rate of reduction of lipid peroxides was found by the pepsin treatment method 2).

Addition of Bovine Bile

The powdered bovine bile used was Ox-Gall of the Difco Co. and was added to the artificial intestinal juice in a ratio of 0, 0.05, 0.10, 0.15, and 0.50 percent for the test.

Table 8

| | Reduction of lipid peroxides (%) |
|---|---|
| Untreated heat-dried dead cells | 100 |
| Pepsin treatment | 95 to 105 |
| Trypsin treatment | 95 to 100 |
| Chymotrypsin treatment | 100 to 115 |
| Insoluble portion of heat-dried dead cells | 100 |
| Addition of bovine bile | |
| 0% | 100 |
| 0.05% | 100 |
| 0.01% | 100 |
| 0.15% | 80 to 95 |
| 0.50% | 80 to 95 |

Example 4

The reduction rate (%) of lipid peroxides was measured as in the previous Examples, for the artificial intestinal juice (2 ml) containing 500 $\mu$g of lipid peroxide and 20 mg of dead cells prepared by treating viable cells of the microorganisms listed in Table 9 with hot water, and freeze-drying. The results are shown in Table 9.

Table 9

| No. | Name of strain | Deposition number | Reduction in lipid peroxides (%) |
|---|---|---|---|
| 1 | Enterococcus faecium AD1060 | FERM BP-1926 (FERM P-9414) | 70 |
| 2 | Enterococcus faecalis AD9001 | FERM BP-297 (FERM P-6625) | 71 |
| 3 | Lactobacillus salivarius AD0003 | FERM BP-1927 (FERM P-9415) | 52 |
| 4 | Saccharomyces cerevisiae 155-77 | FERM BP-1928 (FERM P-9416) | 70 |

12

## Claims

1. A lipid peroxide reducing agent containing as an effective component at least one substance selected from viable cells, dead cells and an insoluble component thereof obtained from Enterococcus faecium AD1060 (FERM BP-1926), Lactobacillus salivarius AD0003 (FERM BP-1927), E. faecalis AD9001 (FERM BP-297), or Saccharomyces cerevisiae 155-77 (FERM BP-1928).

2. A lipid peroxide reducing agent according to claim 1, further containing a pharmaceutically acceptable carrier.

3. A lipid peroxide reducing agent according to claim 1, having a dried form suitable for storage.

4. A novel strain having a lipid peroxide reducing activity, which is selected from Enterococcus faecium AD1060 (FERM BP-1926), Lactobacillus salivarius AD0003 (FERM BP-1927), and Saccharomyces cerevisiae 155-77 (FERM BP-1928).

## Patentansprüche

1. Lipidperoxid verringerndes Mittel aus wenigestens einer Substanz, ausgewählt aus lebensfähigen Zellen, toten Zellen und einem unlöslichen Bestandteil davon als wirksamen Bestandteil, erhalten aus Enterococcus faecium AD 1060 (FERM BP-1926), Lactobacillus salivarius AD 0003 (FERM BP-1927), E. faecalis AD 9001 (FERM BP-297) oder Saccharomyces cerevisiae 155-77 (FERM BP-1928).

2. Lipidperoxid verringerndes Mittel nach Anspruch 1, das zusätzlich einen pharmazeutisch verträglichen Träger enthält.

3. Lipidperoxid verringerndes Mittel nach Anspruch 1, in getrockneter, zur Lagerung geeigneter Form.

4. Ein neuer Zellstamm mit Lipidperoxid verringernder Wirkung, der ausgewählt ist aus Enterococcus faecium AD 1060 (FERM BP-1926), Lactobacillus salivarius AD 0003 (FERM BP-1927) und Saccharomyces cerevisiae 155-77 (FERM BP-1928).

## Revendications

1. Agent réducteur des peroxydes de lipides, contenant, en tant que constituant actif, au moins une substance choisie parmi des cellules viables, des cellules mortes et un composant insoluble de celles-ci, obtenus à partir d'*Enterococcus faecium* AD1060 (FERM BP-1926), *Lactobacillus salivarius* AD0003 (FERM BP-1927), *E. faecalis* AD9001 (FERM BP-297) ou *Saccharomyces cerevisiae* 155-77 (FERM BP-1928).

2. Agent réducteur des peroxydes de lipides selon la revendication 1, contenant en outre un excipient acceptable pharmaceutiquement.

3. Agent réducteur des peroxydes de lipides selon la revendication 1, présentant une forme séchée qui se prête au stockage.

4. Souche nouvelle, ayant une activité de réduction des peroxydes de lipides, choisie parmi *Enterococcus faecium* AD1060 (FERM BP-1926), *Lactobacillus salivarius* AD0003 (FERM BP-1927) et Saccharomyces cerevisiae 155-77 (FERM BP-1928).

# Fig. 1

EP 0 396 744 B1

# Fig. 2

Sample 1 g
   ↓ + 15 ml chloroform-methanol (2:1)

   ↓ Shaken 10 minutes by shaker

   ↓ Centrifuged (3000 rpm 10 minutes)

Supernatent

   ↓ + 3 ml 0.9% saline water

   ↓ Shaken 10 minutes

   ↓ Centrifuged (300 rpm 10 minutes)

Chloroform layer

   ↓ Evaporated to dryness under $N_2$ flow

Residue

   ↓ + 0.2 ml 8.1% sodium lauryl sulfate

   ↓ + 1.5 ml acetic acid-sodium acetate buffer (pH 3.5)

   ↓ + 1.5 ml 0.8% thiobarbituric acid

   ↓ + water (total amount 4 ml)

   ↓ Heated 100°C 30 minutes

   ↓ Cooled

   ↓ + 1 ml water

   ↓ + 5.0 ml n-butanol

   ↓ Shaken

   ↓ Centrifuged (3000 rpm 15 minutes)

Organic solvent layer
    Absorbance at 532 nm measured